Europäisches Patentamt

European Patent Office

Office européen des brevets

(11) Veröffentlichungsnummer: **0 066 901**
**B1**

# EUROPÄISCHE PATENTSCHRIFT

(45) Veröffentlichungstag der Patentschrift:
**18.10.89**

(21) Anmeldenummer: **82200470.1**

(22) Anmeldetag: **19.04.82**

(51) Int. Cl.⁴: **A 61 B 1/06, F 21 S 5/00,**
**F 21 V 9/10**

(54) **Beleuchtungsvorrichtung.**

(30) Priorität: **05.06.81 CH 3710/81**

(43) Veröffentlichungstag der Anmeldung:
**15.12.82 Patentblatt 82/50**

(45) Bekanntmachung des Hinweises auf die Patenterteilung:
**18.10.89 Patentblatt 89/42**

(84) Benannte Vertragsstaaten:
**AT DE FR GB IT SE**

(56) Entgegenhaltungen:
**DE-A- 2 611 318**
**DE-A- 3 007 804**
**DE-B- 1 766 327**
**FR-A- 1 037 779**
**FR-A- 2 405 429**
**GB-A- 663 840**
**US-A- 3 608 547**
**US-A- 3 775 606**

(73) Patentinhaber: **VOLPI AG, Wiesenstrasse 33,**
**CH-8952 Schlieren (CH)**

(72) Erfinder: **Rieder, Peter, Max Müller-Strasse 7,**
**CH-8953 Dietikon (CH)**
Erfinder: **Schürmann, Robert, Seestrasse 691,**
**CH-8706 Meilen (CH)**

(74) Vertreter: **Seifert, Helmut E. et al, RITSCHER & SEIFERT**
**Patentanwälte VSP Kreuzstrasse 82, CH-8032 Zürich**
**(CH)**

## Beschreibung

Die vorliegende Erfindung betrifft eine Vorrichtung zum Beleuchten eines Hohlraums, enthaltend eine Lichtquelle und eine Faseroptik mit Fasern, deren Verteilung im Querschnitt der Eintritts- und der Austrittsfläche ungeordnet ist zum Einleiten des Lichts in den Hohlraum, sowie eine Steuereinrichtung für die Leuchtdichte der Austrittsfläche der Faseroptik, welche Steuereinrichtung als zwischen der Lichtquelle und der Eintrittsfläche der Faseroptik angeordnete, quer zum Lichtweg schieb- oder drehbare und eine keil- bzw. sichelförmige Öffnung aufweisende Blendenscheibe ausgebildet ist.

Vorrichtungen der beschriebenen Art sind beispielsweise aus der DE-A-1 766 327 bekannt und werden u.a. für Spiegelinstrumente zur Untersuchung von Hohlräumen verwendet. Ein bekanntes Beispiel für ein solches Spiegelinstrument ist das Endoskop, das in der Medizin zur direkten Besichtigung von Körperorganen oder in der Technik zum Beobachten schwer zugänglicher Innenräume von Maschinen und Anlagen gebraucht wird. Vorzugsweise enthalten solche Beleuchtungsvorrichtungen zusätzlich ein Wärmeschutzfilter, das zwischen der Lichtquelle und dem Eingang der Faseroptik angeordnet ist und den Eintritt von Wärmestrahlung in der Faseroptik vermeiden soll. Solche Beleuchtungsvorrichtungen werden darum auch als Kaltlichtquellen bezeichnet.

Es gibt verschiedene Möglichkeiten, die Ausleuchtung eines Hohlkörpers durch die Leuchtdichte am Ausgang der Faseroptik zu beeinflussen. Beispielsweise können durch Ändern der Speisespannung oder des Phasenanschnitts der Speisespannung der Lichtstrom oder die Lichtstärke der Lichtquelle gesteuert werden. Dabei wird aber auch die Farbtemperatur der Lichtquelle und folglich auch die Farbe des beleuchteten Objekts verändert, was besonders nachteilig ist, wenn das Bild des Spiegelinstruments fotografiert oder mit einer Fernsehkamera aufgenommen werden soll. Weiter ist es möglich, zwischen der Lichtquelle und dem Eingang der Faseroptik einen verschiebbaren Graukeil anzuordnen. Der Bereich, in dem die Lichtintensität mit einem Graukeil geändert werden kann, ist jedoch begrenzt und ermöglicht insbesondere nicht den unbehinderten Durchtritt des Lichts und damit die maximale Ausleuchtung des Hohlraums. Auch die Verwendung der bekannten mechanischen Blenden ist mit Nachteilen verbunden. Eine Irisblende ändert den Durchmesser des Lichtbündels und damit den Lichteintritts- und den Lichtaustrittswinkel in bzw. aus der Faseroptik, und bei einer Sektoren- oder Fächerblende verbleibt auch bei voller Öffnung ein Sektor im Lichtweg und verhindert, ähnlich wie der Graukeil, die maximal mögliche Ausleuchtung des Hohlraums.

Der vorliegenden Erfindung liegt darum die Aufgabe zugrunde, eine Vorrichtung zum veränderbaren Beleuchten eines Hohlraums zu schaffen, mit der bei voller Öffnung der Lichtfluß von der Lichtquelle nicht behindert und beim Ändern der Beleuchtung weder die Farbtemperatur der Lichtquelle noch der Aperturwinkel des in die Faseroptik ein- oder austretenden Lichtbündels geändert wird.

Erfindungsgemäß wird diese Aufgabe mit einer Beleuchtungsvorrichtung der eingangs definierten Art gelöst, die dadurch gekennzeichnet ist, daß für eine gleichmäßige, von der Größe und der Form des Lichtflecks auf der Eintrittsfläche unabhängige Leuchtdichte auf der gesamten Austrittsfläche, die Faseroptik gemischte Fasern enthält.

Die neue Beleuchtungsvorrichtung ermöglicht, das auf die Eintrittsfläche der Faseroptik fallende Licht stetig zu verändern, ohne die Farbtemperatur oder den Lichteintrittswinkel zu beeinflussen und den maximal möglichen Lichtfluß zu behindern. Durch die Verwendung einer Faseroptik mit gemischten Fasern kann weiter eine gleichmäßige Leuchtdichte der Austrittsfläche der Faseroptik erreicht werden, auch wenn die Fasern in der Eintrittsfläche ungleichmäßig oder nur teilweise beleuchtet werden.

Bei einer bevorzugten Ausführungsform der neuen Vorrichtung ist die Blendenöffnung sichelförmig ausgebildet und die Blendenscheibe um die Mitte des die Symmetrielinie der sichelförmigen Öffnung bildenden Kreises drehbar angeordnet.

Nachfolgend wird eine Ausführungsform der neuen Vorrichtung mit Hilfe der Figuren beschrieben. Es zeigen:

Fig. 1 eine schematische Darstellung der Draufsicht auf die neue Beleuchtungsvorrichtung und

Fig. 2 die Seitenansicht einer Blendenscheibe mit einer sichelförmigen Blendenöffnung.

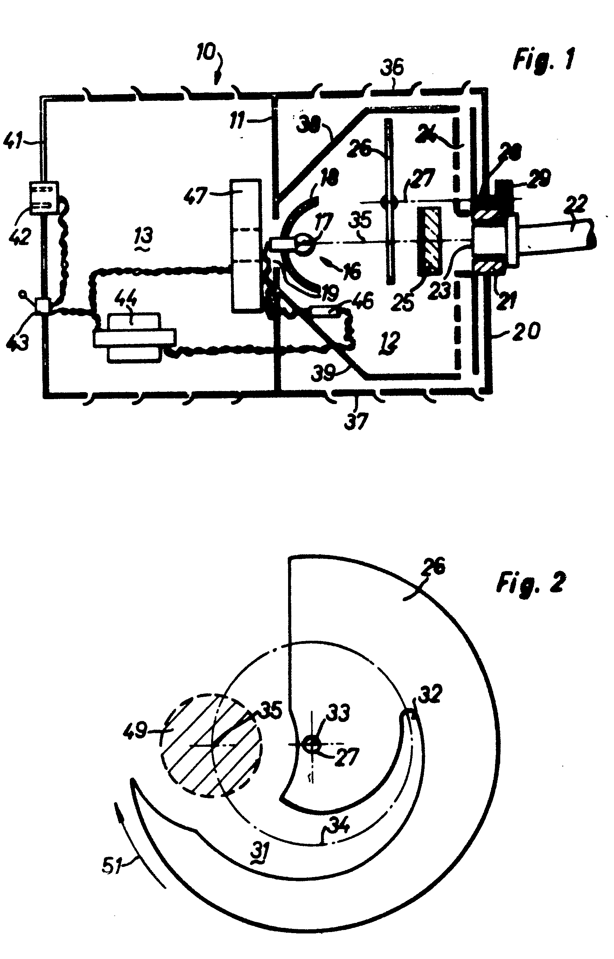

In Fig. 1 ist schematisch die Draufsicht auf eine Beleuchtungsvorrichtung gezeigt, deren Abdeckung abgenommen ist. Die Vorrichtung enthält ein Gehäuse 10, das mittels einer Trennwand 11 in zwei Kammern 12, 13 unterteilt ist. In der ersten Kammer 12 und der Mitte der Trennwand benachbart ist eine Lichtquelle 16 angeordnet. Diese Lichtquelle enthält einen Glaskolben 17 mit einer Glühwendel und einen Kaltlichtspiegel 18, der für Wärmestrahlung durchlässig ist. Der Sockel der Lichtquelle ist in eine Öffnung 19 in der Trennwand eingesetzt. Der Durchmesser dieser Öffnung ist wesentlich größer als der Durchmesser des Lampensockels, um das Durchströmen von Kühlluft zu ermöglichen.

In der Vorderwand 20 des Gehäuses und praktisch der Lampe gegenüberliegend ist ein Anschlußstück 21 für eine Faseroptik 22 befestigt. Die Faseroptik kann in das Anschlußstück eingeschraubt, eingesteckt oder mit einem Bajonettverschluß gehaltert werden. Der Innenseite der Vorderwand und der Eintrittsfläche 23 der Faseroptik benachbart ist ein zur Führung von Kühlluft vorgesehener Kühlkörper 24 angeordnet. Weiter ist vor der Eintrittsfläche der Faseroptik ein Interferenzfilter 25 eingebaut mit einer der Lichtquelle zugewandten IR-Strahlung reflektierenden Beschichtung. Der Kaltlichtspiegel 18 an der Lichtquelle 16 und das Interferenzfilter 25 vermindern den Anteil der IR-Strahlung in dem auf die Faseroptik auftreffenden und von dieser weitergeleiteten Licht. Damit soll insbesondere eine Erwärmung der Faseroptik vermieden werden, weil bei länger einwirkender Wärme das zwischen den einzelnen Fasern befindliche Klebmittel erweichen und die Faseroptik nachteilig beeinflußt oder gar zerstört werden kann.

Im Weg des Lichtbündels 49 mit der Bündelachse 35 ist zwischen der Lichtquelle 16 und dem Interferenzfilter 24 weiter eine Blende 26 eingebaut. Die Blende ist als kreisförmige Scheibe ausgebildet und auf einer Drehachse 27 befestigt. Die Drehachse ist durch eine Führungsöffnung 28 in der Vorderwand des Gehäuses geführt, und an ihrem freien Ende ist ein zum manuellen Betätigen vorgesehener Drehknopf 29 befestigt.

Wie in Fig. 2 gezeigt ist, weist die Blendenscheibe einen sichelförmigen Schlitz 31 auf, der sich von einem sehr schmalen inneren Ende 32 symmetrisch zu einer kreisbogenförmig um den Mittelpunkt 33 verlaufenden Symmetrielinie 34 erweitert. Der Abstand der Symmetrielinie vom Mittelpunkt 33 der Blendenscheibe entspricht dem Abstand der Drehachse 27 der Blendenscheibe von der Achse 35 des von der Lichtquelle zur Faseroptik gerichteten Lichtbündels.

Die Seitenwände 36, 37 der ersten Kammer weisen Luftschlitze auf, und die Lichtquelle 16, die Blendenscheibe 26 sowie das Filter 25 sind von zwei Leitwänden 38, 39 für die Kühlluft eingeschlossen.

In der Rückwand 41 der zweiten Kammer 13 sind eine Steckbuchse 42 zum Anschluß der Beleuchtungseinrichtung an das elektrische Netz und ein Schalter 43 befestigt. In der zweiten Kammer befinden sich ein Transformator 44, dessen Sekundärwicklung die Speisespannung für die Lichtquelle 16 liefert, sowie ein der Öffnung 19 in der Trennwand 11 benachbart angeordneter Tangentiallüfter 47. Zum Schutz der Lichtquelle gegen Überhitzen ist die Stromleitung vom Transformator 44 zur Lichtquelle 18 durch die erste Kammer 12 geführt, wo in unmittelbarer Nachbarschaft der Lichtquelle eine Thermosicherung 46 vorgesehen ist.

Es versteht sich, daß bei geeignet gewählter Form der sichelförmigen Öffnung in der Blendenscheibe eine lineare oder eine logarithmische oder sonst eine erwünschte Abhängigkeit der leuchtdichte der Austrittsfläche der Faseroptik vom Drehwinkel der Blendenscheibe erreicht werden kann.

Bei der Verwendung der neuen Vorrichtung wird das von der Glühwendel im Glaskolben 17 abgestrahlte Licht vom Kaltlichtspiegel 18 in ein praktisch parallel auf die Eintrittsfläche der Faseroptik 23 gerichtetes Lichtbündel 49 gewandelt. Wie bereits oben angegeben ist, ist der Kaltlichtspiegel für IR-Strahlung von der Beschichtung und dem Interferenzfilter 25 zum überwiegenden Teil reflektiert bzw. ausgelöscht, so daß das in die Faseroptik 22 eintretende Licht praktisch keine Wärmestrahlung enthält. Der Querschnitt des auf die Eintrittsfläche der Faseroptik 23 auftreffenden Lichtbündels kann durch Drehen der Blendenscheibe 26 verändert werden. Wenn die Blendenscheibe die in Fig. 2 gezeigte Form und Ausnehmung und das von der Lichtquelle erzeugte Lichtbündel den mit gestrichelten Linien gezeichneten Querschnitt 49 aufweisen, dann wird beim Drehen der Blendenscheibe in der Richtung des Pfeils 51 der Querschnitt des Lichtbündels in horizontaler Richtung verkleinert, während er in der vertikalen Richtung unverändert bleibt. Das hat zur Folge, daß die Beleuchtungsstärke auf der Eintrittsfläche 23 des Lichtbündels verringert wird, während der Eintrittswinkel des in der Querschnittsfläche verkleinerten Lichtbündels mindestens in einer Richtung unverändert bleibt.

Für eine praktisch erprobte Ausführungsform der neuen Vorrichtung wurde als Lichtquelle eine Halogen-Kaltlicht-Spiegellampe verwendet. Der Durchmesser der Blendenscheibe betrug 90 mm, der Durchmesser der kreisförmigen Symmetrielinie für die sichelförmige Öffnung 50 mm. Die sichelförmige Öffnung erstreckte sich über einen Winkel von etwa 180° und erweiterte sich von einer quer zur Symmetrielinie gemessenen kleinsten Breite von 2 mm bis zu einer größten Breite von 20 mm. Die verwendete Faseroptik konnte an die vorgegebene Verwendung der Vorrichtung angepaßt werden. Zwei gebräuchliche Faseroptiken hatten einen Durchmesser von 13 mm und enthielten 62 000 Glasfasern mit je 50 μ Durchmesser oder 32 000 Glasfasern mit je 70 μ Durchmesser. Zwei andere brauchbare Faseroptiken hatten einen Durchmesser von nur 0,5 mm und enthielten 95 Glasfasern mit je 50 μ Durchmesser bzw. 50 Glasfasern mit je 70 μ Durchmesser.

Die durch die Lufteintrittschlitze in den Seitenwänden 36, 37 der ersten Kammer 12 eintretende Kühlluft wird von den Leitwänden 38, 39 zum Einlaß des Kühlkörpers 24 geführt und strömt von dort über die Eintrittsfläche der Faseroptik und das Filter 25 zur Lichtquelle 16. Die erwärmte Kühlluft wird dann vom Tangentiallüfter 47 durch die Öffnung 19 in der Trennwand 11 in die zweite Kammer 13 gesaugt und verläßt diese zweite Kammer durch die Lüftungsschlitze in den Kammerwänden.

Es versteht sich, daß das beschriebene Ausführungsbeispiel der neuen Vorrichtung auf vielerlei Weise abgeändert werden kann und insbesondere anstelle der beschriebenen Blendenscheibe mit einer sichelförmigen Öffnung auch eine Scheibe verwendet werden kann, die eine keilförmige Öffnung aufweist und in der Richtung der Mittellinie des Keiles verschiebbar ist.

Die gesamte Vorrichtung kann mit handelsüblichen Bauteilen hergestellt werden, weshalb auf eine detaillierte Beschreibung dieser Bauteile und deren gegenseitige Anordnung verzichtet wird.

**Patentanspruch**

Vorrichtung zum Beleuchten eines Hohlraums, enthaltend eine Lichtquelle (16) und eine Faseroptik (22) mit Fasern deren Verteilung im Querschnitt der Eintritts- und der Austrittsfläche ungeordnet ist zum Einleiten des Lichts in den Hohlraum, sowie eine Steuereinrichtung für die Leuchtdichte der Austrittsfläche der Faseroptik, welche Steuereinrichtung als zwischen der Lichtquelle (16) und der Eintrittsfläche (23) der Faseroptik (22) angeordnete, quer zum Lichtweg schieb- oder drehbare und eine keil- bzw. sichelförmige Öffnung aufweisende Blendenscheibe (26) ausgebildet ist, dadurch gekennzeichnet, daß für eine gleichmäßige, von der Größe und der Form des Lichtflecks auf der Eintrittsfläche unabhängige Leuchtdichte auf der gesamten Austrittsfläche, die Faseroptik gemischte Fasern enthält.

## Claim

An apparatus for illuminating a cavity, said apparatus including a light source (16) and a fibre optics (22) comprising fibres in unordered cross-sectional distribution over the entry and exit face for conducting light into said cavity, and a device for controlling the density of illumination at the exit face of the fibre optics, said device for control being constituted as a slideable or turnable aperture disk (26) having a wedge-shoped or sickle-shaped aperture, said aperture disk being arranged between said light source (16) and said entry face (23) of said fibre optics, characterized in that the fibre optics includes mixing fibres for achieving a uniform density of illumination over the entire exit face independent from size and shape of the light spot on the entry face.

## Revendication

Dispositif pour éclairer une cavité, comprenant une source lumineuse (16) et, pour introduire la lumière dans la cavité, und optique sur fibres (22) ayant des fibres dont la distribution est sans ordre dans la section des surfaces d'entrée et de sortie, ainsi qu'un dispositif de commande de la luminance de la surface de sortie de l'optique sur fibres, ce dispositif de commande étant formé par un disque diaphragme (26) qui présente une ouverture en forme de coin ou de croissant et est disposé entre la source lumineuse (16) et la surface d'entrée (23) de l'optique sur fibres (22), et qui peut être déplacé ou pivoté en direction transversale par rapport au trajet optique, caractérisé en ce que l'optique sur fibres comprend des fibres mélangées en vue d'obtenir une luminance égale sur la surface de sortie entière, indépendamment de la grandeur et de la forme du spot lumineux sur la surface d'entrée.

Fig. 1

Fig. 2